# EUROPEAN PATENT APPLICATION

(11) **EP 0 645 160 A1**
(43) Date of publication of application: **29.03.1995**
(21) Application number: 93201855.9
(22) Date of filing: 25.06.1993
(51) Int. Cl.: A61M 36/12, A01K 11/00

(54) **Injector and object to be injected by the injector**

(71) Applicant: TEXAS INSTRUMENTS INCORPORATED, Dallas Texas 75265 (US); Texas Instruments Holland B.V., NL-7600 AA Almelo (NL)
(72) Inventor: Benning, Franciscus Hendrikus Cornelis, NL-7609 JS Almelo (NL); Niezink, Herman, NL-7642 GP Wierden (NL); Kruit, Leo Karel, NL-3607 MD Maarssen (NL)
(74) Representative: de Bruijn, Leendert C.

(57) **Abstract**

Injector for introducing an object such as a transponder (2) in a livin being. Through the arrangement of a sharpened tip (4) on the transponder the opening in the skin of the living being is realized by the transponder itself and not by any sharpened needle point of the injector tube (5). This means that the circumferential end edge of the transponder can be blunt. The tip of the transponder comprises a bio-compatible, preferably bio-degradable, material in which possibly a medication is provided.

## Description

The invention relates to an injector for introducing an object, such as a transponder, in a living being, comprising a housing to which an injector tube is connected for receiving and guiding the object.

Such an injector is generally known in the art. Objects, such as transponders are injected in animals, e.g. for identification purposes. The opening in the skin of the animal is realized in that the tip of the injector tube is sharpened. This generally means that the injector is a hollow needle of which the end face is sharpened to provide an opening.Because of that during non-use there is a substantial danger that the operators using the injector can hurt themselves or others through the tip of the needle. To prevent these problems it is proposed that the needle is retractable. However, due to the dirt and the nature of the skin of several animals high requirements are set to the material from which the needle tip is realized. Often wear takes place after a relative low number of transponders has been injected such that the force to protrude the skin will increase and the accuracy of placing of the transponder will decrease. Furthermore there is the problem of cross contamination between animals through the needle tip.

The invention aims to provide an improved injector which can be used together with a new transponder to avoid the drawbacks relating to the presence of a sharp needle tip.

According to the invention this is realized in that the free extremity of the injector tube comprises a blunt circumferential edge. The invention also relates to an transponder comprising an elongated body.

According to the invention this object is characterized in that in front of the body a sharpened tip is provided.

The invention is based on the idea that the object to be injected itself is provided with a sharp tip so that it is not longer necessary that an opening is provided in the living being with the injector tube. The injector tube only acts as guide for the transponder to be introduced. This means that the free extremity of the injector tube can be blunt so that in the position of non use it is completely safe and no separate costly measures have to be taken to prevent that this end contacts the operators or other persons. On the other hand such a device is extremely simple. The tip of the transponder enters the living being together with the transponder member and is only used a single time. This means that the danger of cross contamination is reduced whilst abrasive conditions during introduction of the tip do not have any effect on the service life of this tip because it is for single use only.

According to a preferred embodiment of the invention the injector tube is removable from the casing of the injector. This means that in a simple way a new simple injector tube can be placed on the casing which further reduces the danger of cross contamination.

To increase the acceptance of the transponder in the injector liquid dosing means are provided connected to the injector tube. During or before each introduction of the transponder in the living being a liquid can be metered preventing infection or a disinfecting the transponder during its introduction. Of course this fluid can contain any other kind of medication.

The free extremity of the injector tube cannot only function as guiding for the transponder but also acts as support. In the last case preferably a transition area is provided between the rear end of the tip and the front end of the transponder member. The blunt end surface of the extremity of the injector tube rests on this abutment face and forces exerted on the grip or housing of the injector are transmitted through this extremity to the tip of the transponder whilst the vulnerable transponder member being received in the injector tube is not subjected to these forces and protected against damage during introduction.

To facilitate introduction of the transponder the tip is provided with at least two external ribs. These external ribs make a incision such that skin flaps result and punching out or loosening of parts of the skin is prevented during introduction of the transponder decreasing the danger of infection of the animal during healing of the wound.

To further prevent infections and rejection of the transponder the tip is preferably fabricated from a material being bio-compatible with the living being. An example for such bio-compatible material is bone material. After machining to the desired shape and disinfecting e.g. by boiling it has been shown that this material is very suitable for use as tip material. Bone material is furthermore a biode-degradable material. Through sterilizing by boiling only the harder part of the bone material will remain and the marrow parts will disappear. This harder part will disintegrate after introduction into the animal. Except from preparing of a tip by machining from bone material it is also possible to first grind such bone material and to press-shape the related tip. Because bone material is a natural material it is easily accepted by animals. It has been observed that after several weeks of introduction at least the sharpest parts of the tips will be dissolved. This prevents the transponder from wandering through the animal. The abutment surface described above between the tip and the transponder member also acts as barb surface preventing rearward movement of the transponder after introduction. Of course a medication can be added to the tip such as a disinfectant. In view of further miniaturization of the transponder member it is quite possible that the tip is also body for this transponder member. This means that in the tip an opening is provided in which the transponder member is received.

The invention will be further elucidated with reference to a preferred embodiment which is described below and illustrated in the enclosed drawing, wherein:
Fig. 1 shows an injector according to the invention together with a transponder;
Fig. 2 shows more detailed the transponder according to the invention;
Fig. 3 shows the transponder according to the invention introduced subcuntaneous in an animal; and
fig. 4 shows a further embodiment of the transponder according to the invention.

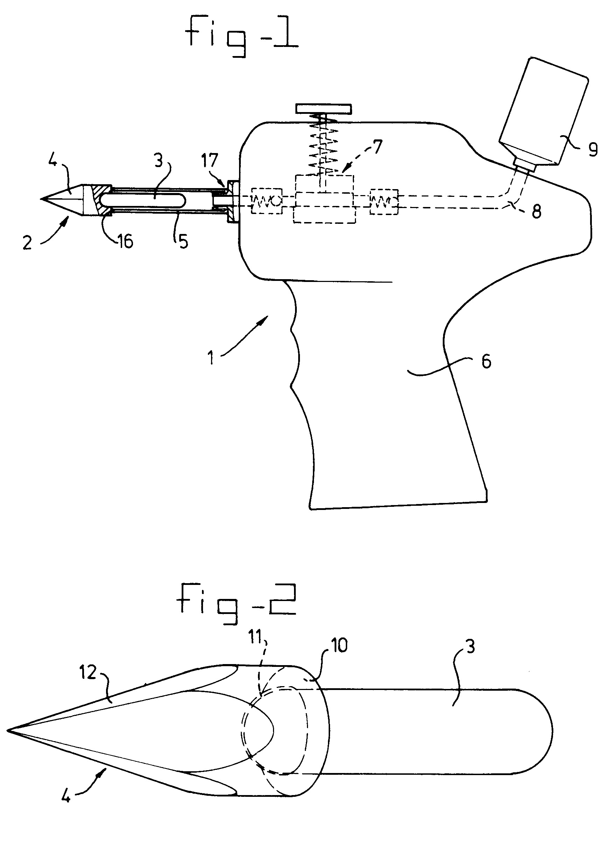

In Fig. 1 the injector according to the invention is generally indicated with 1 whilst the transponder has the general reference number 2. Transponder 2 comprises a transponder member 3 as is generally known in the art and a transponder tip 4. This transponder member can be any transponder having either a glass or plastic housing and is usally of elongated shape having rounded extremities. Tip 4 is provided with four cutting ribs 12 as is clear from Fig. 2. Connection between tip 4 and transponder member 3 is preferably realized by a layer 11 of glue. If tip 4 is realized from bone material, layer 11 obviously has good adhesion properties with regard to this material and the material from which the transponder housing is prepared.

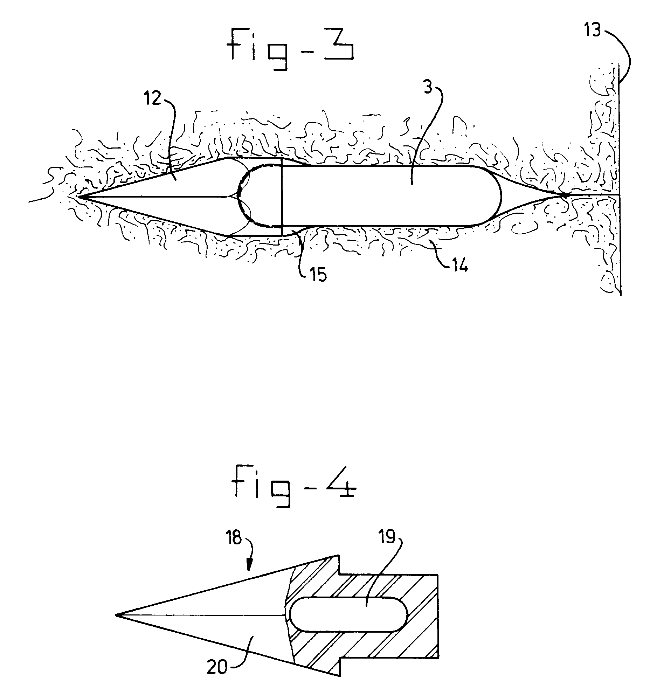

As is clear from Fig. 2 the outer circumference of the non-sharpened end of tip 12 is somewhat larger than the outer circumference of transponder member 3 adjacent thereto. This means that an abutment face 10 is realized. This abutment face 10 is dimensioned such that the blunt extremity of injector tube 5 (see fig.1) bears against this abutment face. Forces during introduction are directly transferred from the injector tube to the tip and not through the transponder member 3 as with prior art devices wherein a push rod is used inside injector tube 5. Injector tube 5 is connected to housing 6 through a quick connecting means 17. This means that injector tube 5 can be easily replaced by a fresh tube and because the requirements set to the free extremities of the injector tube 5 are relatively low the costs for producing such tube are also relatively low. The interior of the hollow tube 5 is connected to pump 7 which is in turn connected to reservoir 9 through conduit 8. This reservoir 9 contains a disinfectant agent which can be introduced by pumping into injector tube 5 and so around transponder member 3. During introduction the single use tip 4 cuts an opening in skin 13 of the animal (Fig. 3). Because of the presence of four ribs 12 two cross cuts are realized in skin 13 resulting in four flaps which are bent inwardly into the animal and after introduction of the transponder move outwardly again such that no parts of the skin will be introduced in the animal. In Fig. 3 the position of the transponder in tissue 14 is shown. Because of the presence of abutment surface 10 on position 15 in tissue 14 a restriction will grow after introduction of the transponder preventing the transponder from moving back in the direction to the skin. Because of the bio-degradable nature of tip 4 the most exposed part of it being the front end of the tip will "erode" relatively fast such that movement in the leftward direction of Fig. 3 is also prevented. It has been observed that after introduction the end of the tip irritates the tissue of the animal such that its rejection mechanism will become active around the wound and provide an encapsulation. However, before the rejection mechanism is able to reject the transponder the free extremity of the tip is degraded considerable and will be kept in its position. The connection between tip and transponder member is such that if a non-axial load is inserted it will break through the layer 11 of glue.

The bone material tip can be produced by first grinding of bones and afterwards boiling during at lest 24 hours. During boiling the marrow material will substantially disintegrate and the harder part of the bone remains. After that disinfectant, such as iodine, is added and the bone material is pressed to the tip shape. It is glued with bone cement to the transponder member.

It has been observed that after introduction into a living being after about two weeks the most sharpened parts are disappeared and the remaining tip has the properties of cartilage.

In Fig. 4 a further embodiment is shown wherein the transponder member 19 is further reduced in size such that it can easily be received in body 20 of the transponder 18. Tip and body are made from the same material.

Although the invention is described above relating to a preferred embodiment it will be clear for the person skilled in the art that many amendments can be made without leaving the scope of protection as defined in the appended claims.
It is e.g. possible to realize the tip from any bio-compatible material not being bone material.

## Claims

1. Injector (1) for introducing an object, such as a transponder (2), in a living being, comprising a housing (6) to which an injector tube (5) is connected for receiving and guiding the object, characterized in that the free extremity of the injector tube comprises a blunt circumferential edge (16).

2. Injector according to claim 1, wherein the injector tube is removable from the housing.

3. Injector according to one or more of the preceding claims, wherein liquid dosing means (7-9) are provided connected to the injector tube.

4. Object to be injected in a living being, such as a transponder, comprising an elongated body, characterized in that in front of the body a sharpened tip (4) is provided.

5. Object according to claim 4, wherein the tip is provided with at least two external ribs (12).

6. Object according to claim 4 or 5, wherein at least the tip comprises a material being bio-compatible with the living being.

7. Object according to one of the claims 4-6, wherein the bio-compatible material comprises bone material.

8. Object according to one of the claims 4-7, wherein the bio-compatible material comprises biodegradable material.

9. Object according to one of the claims 4-8, wherein the tip comprises a medication.

10. Object according to claim 9, wherein the medication comprises a disinfectant.

11. Object according to one of the claims 4-10, wherein a transition area (10) is provided between the rear end of the tip and the front end of the transponder, wherein the outer circumference of the tip at its rear ends extends beyond the outer circumference of the front end of the transponder.

12. Object according to claim 11, wherein the transition area defines an abutment surface.

13. Object according to one of the claims 4-12, wherein the tip comprises a cavity to receive a transponder member (19).
